# EUROPEAN PATENT APPLICATION

(11) **EP 1 872 782 A1**
(43) Date of publication of application: **02.01.2008**
(21) Application number: 06731845.1
(22) Date of filing: 14.04.2006
(51) Int. Cl.: A61K 31/409, A61K 41/00, A61P 9/14, A61P 35/00, C07D 487/22

(54) **DRUG FOR TREATING OR DIAGNOSING VASCULAR LESION IN THE SKIN OR THE SUBCUTANEOUS SOFT TISSUE CAUSED BY LIGHT IRRADIATION**

(30) Priority: 14.04.2005 JP 2005117183
(71) Applicant: Ohshiro, Takafumi, Tokyo 160-0016 (JP)
(72) Inventor: OHSHIRO, Takafumi, Shinjuku-ku, Tokyo 1600016 (JP); OHSHIRO, Toshio, Shinjuku-ku, Tokyo 1600016 (JP)
(74) Representative: Wytenburg, Wilhelmus Johannes
(86) International application number: PCT/JP2006/307909
(87) International publication number: WO 2006/112379

(57) **Abstract**

A novel drug for treating a vascular lesion in skin or subcutaneous soft tissue by light irradiation is disclosed, which is effective for treating a lesion in a deep portion and yet which is free from destruction of normal cells in the skin surface, and with which the shading time after treatment is short, in the treatment of a vascular lesion in skin or subcutaneous soft tissue by light irradiation. The drug for treating a vascular lesion in skin or subcutaneous soft tissue by light irradiation comprises as an effective ingredient mono-L-aspartyl chlorin e6 or mono-L-glutamyl chlorin e6, or a pharmaceutically acceptable salt thereof.

## Description

### Technical Field

The present invention relates to a drug for treating or diagnosing a vascular lesion in skin or subcutaneous soft tissue by light irradiation. More particularly, the present invention relates to a drug for treating a vascular lesion in the skin or subcutaneous soft tissue by light irradiation, the drug being a photosensitizer which is administered to a patient when the vascular lesion in the skin or subcutaneous soft tissue is treated or diagnosed by light irradiation, which photosensitizer is excited by the light to selectively destruct the target cells; and to a diagnostic agent by which the two-dimensional and further the three-dimensional extent of the lesion may be diagnosed.

### Background Art

The therapy for cutaneous vascular lesions (capillary hemangioma, strawberry hemangioma, cavernous hemangioma and the like), which is now being firstly selected is the therapy by dye laser. However, the therapeutic effect by dye laser to a lesion at a deep portion is limited, and the therapy must be performed frequently, which imposes heavy temporal, psychological and economical burdens to patients, that is problematic.

On the other hand, photodynamic therapy (PDT) has drawn attention as a selective therapy for cancers. This therapy comprises administering a photosensitizer which has an affinity to tumors and which is accumulated to newly formed blood vessels; irradiating a tumor tissue with a laser to cause a photochemical reaction so as to subject the tumor tissue to necrosis. The therapeutic effect is due to the oxidation by the singlet oxygen produced by the photochemical reaction between the laser light and the photosensitizer.

As the photosensitizer for PDT, Polypharma Sodium which is a porphyrin-related compound is known. However, this compound has a problem in that a shading time of at least 4 weeks is necessary after the therapy. That is, since the photosensitizer reacts with light to destruct the cells, the patient must prevent the body from exposure to light for at least 4 weeks. Otherwise, the skin is inflamed due to hyperphotosensitivity. Therefore, the patient must stay in a dim room for at least 4 weeks, so that the patient cannot live a normal daily life.

On the other hand, it is known to use mono-L-aspartyl chlorin e6 or mono-L-glutamyl chlorin e6, or a pharmaceutically acceptable salt thereof, which are porphyrin-related compounds, as a photosensitizer for the photodynamic therapy, for the therapy of lung cancer (Patent Literature 1) or occlusion of newly formed blood vessels (Patent Literature 2 and Patent Literature 3) (the mono-L-aspartyl chlorin e6 used in Patent Literature 2 and Patent Literature 3 are different in the esterified sites). However, it is not known at all to apply these phososensitizer to the therapy of cutaneous vascular lesions by laser irradiation.

On the other hand, no method is known for diagnosing the two-dimensional and further the three-dimensional extent of a vascular lesion in the skin or subcutaneous soft tissue, which lesion cannot be observed from the skin surface. If the two-dimensional and further the three-dimensional extent of a vascular lesion can be diagnosed, it is advantageous to therapies no matter which therapeutic method is employed.

Patent Literature 1: WO 98/19677 A
Patent Literature 2: WO 01/60360 A
Patent Literature 3: JP 9-71531 A
Patent Literature 4: JP 8-243181 A

### Disclosure of the Invention

### Problems to be Solved by the Invention

An object of the present invention is to provide a novel drug for treating a vascular lesion in skin or subcutaneous soft tissue by light irradiation, which is effective for treating a lesion in a deep portion and yet which is free from destruction of normal cells in the skin surface, and with which the shading time after treatment is short, in the treatment of a vascular lesion in the skin or subcutaneous soft tissue by light irradiation. Another object of the present invention is to provide a diagnostic agent by which the two-dimensional and further the three-dimensional extent of lesion may be diagnosed, which lesion is a vascular lesion in skin or subcutaneous soft tissue, which cannot be observed from the skin surface.

### Means for Solving the Problems

The present inventors intensively studied to discover that by treating a cutaneous vascular lesion by light irradiation using mono-L-aspartyl chlorin e6 or mono-L-glutamyl chlorin e6, or a pharmaceutically acceptable salt thereof, which are chlorin-based drugs, as the photosensitizer for photodynamic therapy, the vascular endothelial cells in the dermis disappear and the cutaneous vascular lesion is cured, while the epidermis cells are retained in normal state, thereby completing the present invention. It is known that mono-L-aspartyl chlorin e6, mono-L-glutamyl chlorin e6 and pharmaceutically acceptable salts thereof emit fluorescence having a peak at about a wavelength of 670 nm. The present inventors inferred that the two-dimensional and further the three-dimensional extent of a vascular lesion in the skin or subcutaneous soft tissue may be diagnosed by utilizing the fluorescence (that is, from the area from which the fluorescence is emitted, the breadth of the lesion can be diagnosed, and from the intensity of the fluorescence, the depth of the lesion can be diagnosed), thereby completing the present invention.

That is, the present invention provides a drug for treating a vascular lesion in skin or subcutaneous soft tissue by light irradiation, the drug comprising as an effective ingredient mono-L-aspartyl chlorin e6 or mono-L-glutamyl chlorin e6, or a pharmaceutically acceptable salt thereof. The present invention also provides a use of mono-L-aspartyl chlorin e6 or mono-L-glutamyl chlorin e6, or a pharmaceutically acceptable salt thereof for the production of a drug for treating vascular lesion in skin or subcutaneous soft tissue by light irradiation. The present invention further provides a method for treating a vascular lesion in skin or subcutaneous soft tissue by light irradiation, the method comprising the steps of administering to a patient suffering from the vascular lesion in skin or subcutaneous soft tissue mono-L-aspartyl chlorin e6 or mono-L-glutamyl chlorin e6, or a pharmaceutically acceptable salt thereof; and then irradiating the vascular lesion with light.

The present invention also provides a diagnostic agent for a vascular lesion in skin or subcutaneous soft tissue, comprising as an effective ingredient mono-L-aspartyl chlorin e6 or mono-L-glutamyl chlorin e6, or a pharmaceutically acceptable salt thereof. The present invention further provides a use of mono-L-aspartyl chlorin e6 or mono-L-glutamyl chlorin e6, or a pharmaceutically acceptable salt thereof for the production of a diagnostic agent for a vascular lesion in skin or subcutaneous soft tissue. The present invention still further provides a method for diagnosing a vascular lesion, the method comprising the steps of administering to a patient suffering from the vascular lesion in skin or subcutaneous soft tissue mono-L-aspartyl chlorin e6 or mono-L-glutamyl chlorin e6, or a pharmaceutically acceptable salt thereof; then irradiating the vascular lesion with light; and measuring fluorescence from the vascular lesion.

### Effects of the Invention

By the present invention, a drug for treating a vascular lesion in the skin or subcutaneous soft tissue by light irradiation, which is effective for treating a lesion in a deep portion and yet which is free from destruction of normal cells in the skin surface, and with which the shading time after treatment is short, in the treatment of a vascular lesion in the skin or subcutaneous soft tissue by light irradiation, was first provided. As will be concretely described in the Examples below, by using the drug for treating a vascular lesion in the skin or subcutaneous soft tissue by light irradiation according to the present invention, the decoloration of the cutaneous vascular lesion irradiated with light is prominent, disappearance of vascular endothelial cells is observed, and yet the continuity of stratum corneum epidermidis is maintained and the stratum basale epidermidis is also conserved. Further, since the shading time of this drug may be about 2 to 3 days, the constraints of the patient's daily life are small. Therefore, the drug for treating a vascular lesion in the skin or subcutaneous soft tissue is very effective for the therapies of vascular lesions in the skin or subcutaneous soft tissue, represented by capillary hemangioma, strawberry hemangioma, cavernous hemangioma, teleangiectasia and arteriovenous malformation. Further, by the present invention, a diagnostic agent for a vascular lesion in the skin or subcutaneous soft tissue was first provided, by which the two-dimensional and further the three-dimensional extent of the vascular lesion in the skin or subcutaneous soft tissue can be diagnosed, which lesion cannot be observed from the skin surface. The mechanism of the therapy and diagnosis by the present invention is not to irradiate the drug as a target after waiting for the accumulation of the drug in ever-growing abnormal cells or in the vicinity thereof, but the lesion at which the amount of the existing blood, and in turn, the amount of the drug, is relatively larger than in the vicinity thereof due to the vasodilation of the blood vessels *per se,* is irradiated with light. Therefore, the therapy can be performed if the drug is uniformly distributed in the entire body of the patient, and so the light irradiation may be conducted almost immediately after the administration of the drug, burden to wait is less for both the physician and the patient.

### Best Mode for Carrying out the Invention

Mono-L-aspartyl chlorin e6, mono-L-glutamyl chlorin e6 and pharmaceutically acceptable salts thereof, as well as the production processes thereof, are known and described in Patent Literature 1 and Patent Literature 2. The chemical structure is shown in Formula (A) below.

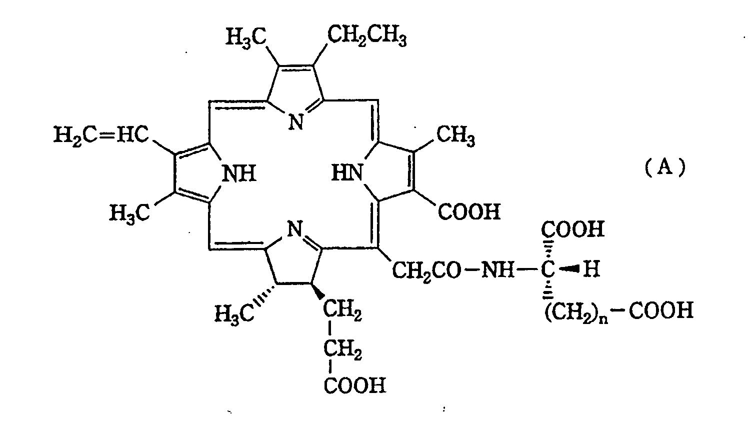

In Formula (A), n is an integer of 1 or 2. When n is 1, the compound is mono-L-aspartyl chlorin e6, and when n is 2, the compound is mono-L-glutamyl chlorin e6. All or a part of the 4 carboxyl groups may be in the form of a pharmaceutically acceptable salt, and alkaline metal salts such as sodium salt are preferred. Tetrasodium salt is especially preferred. Tetrasodium salt of mono-L-aspartyl chlorin e6 (talaporfin sodium) is commercially available for the treatment of lung cancer, and an apparatus for radiating diode laser having a wavelength of 664.5 nm which is the wavelength of its absorption maximum is also commercially available.

The diseases to which the drug of the present invention is applied are vascular lesions in the skin or in the subcutaneous soft tissue. Preferred examples thereof include, but not limited to, capillary hemangioma, strawberry hemangioma, cavernous hemangioma, teleangiectasia and arteriovenous malformation. Among these diseases, preferred diseases are cutaneous vascular lesions, and especially preferred examples include capillary hemangioma, strawberry hemangioma and cavernous hemangioma. These vascular lesions in the skin or in the cutaneous soft tissue are benign hemangioma, and do not at all or do not almost accompany neovascularization.

The preferred administration routes of the drug of the present invention are parenteral administration routes such as intravenous injection, subcutaneous injection intramuscular injection and the like. Although the dose is appropriately selected depending on the degree of the vascular lesion in the skin or subcutaneous soft tissue, and so on, as described later, the present inventors discovered that the dose per one photodynamic therapy is about 0.1 mg to 1.0 mg, preferably about 0.1 mg to 0.5 mg per 1 kg of bodyweight. The drug may be formulated in the form of an injection solution or the like by a conventional method. For example, the formulation may be a solution prepared by dissolving the drug in physiological buffer to a concentration of about 5 mg/ml to 50 mg/ml.

After administering the drug according to the present invention, the site of the vascular lesion in the skin or in the subcutaneous soft tissue is irradiated with light. In case of the vascular lesion in the subcutaneous soft tissue, after exposing the affected area by surgery and after administering the above-described drug during the surgery, the affected area is irradiated with light. It is preferred to carry out the light irradiation from immediately after the administration of the drug to 1 hour from the administration in view of the above-described mechanism. The wavelength of the light is preferably 664.5nm, 609.5nm, 507.3nm or 411.0nm, or in the vicinity (±5 nm) thereof, at which the drug has an absorption maximum. Among these wavelengths, employing one which can reach deeper portion at a lower output power is preferred because the damage to the normal skin surface can be made small. Thus, the wavelength of the light to be radiated is most preferably the long one, that is, 664.5 nm or the vicinity (±5 nm) thereof. The light may be any light as long as it contains the light component, and laser light is preferred. However, another light source such as light emitting diode (LED) may also be employed. In cases where the laser is used, the power density thereof is preferably 20 mW/cm² to 200 mW/cm², and the energy density is preferably 25 J/cm² to 100 J/cm². The light such as laser or the like may be either a continuous light or pulsed light. Using a pulsed light is preferred because the damage to the normal skin surface can be made small.

It is known that mono-L-aspartyl chlorin e6, mono-L-glutamyl chlorin e6 and pharmaceutically acceptable salts thereof emit fluorescence having a peak at about a wavelength of 670 nm (Patent Literature 4). Utilizing this fluorescent property, the two-dimensional and further the three-dimensional extent of the vascular lesion in the skin or in the subcutaneous soft tissue can be diagnosed. That is, although the two-dimensional and further the three-dimensional extent of the vascular lesion in the skin cannot be known by visual observation from the skin surface, these may be diagnosed by measuring the above-described fluorescence. That is, since fluorescence is emitted from the vascular lesion by irradiating the vascular lesion with an excitation light, the two-dimensional and further the three-dimensional extent of the lesion may be diagnosed by measuring the fluorescence. As the excitation light, as in the case of therapy, the light having a wavelength of 664.5 nm or the vicinity thereof (±5 nm) is preferred. Therefore, the same light irradiation apparatus as used in the therapy can be used.

The present invention will now be described concretely by way of Examples thereof. However, the present invention is not restricted to the Examples below.

### Example 1

### Experiments Using Model Animals (cocks) of Cutaneous Vascular Lesion

### Methods

Animals Used: cocks (bodyweight: 2.5 to 3.5 kg) of 22 to 24 weeks old
Photosensitizer: talaporfin sodium
Laser Used: Panalas6405, diode laser, wavelength: 664.5 nm ± 2 nm, output power: 75-500 mW

As the model of PDT to cutaneous vascular lesion (capillary hemangioma), cock's combs were used. Cock's comb is recognized as a model of cutaneous vascular lesion (capillary hemangioma) in this field. To each cock of 22 to 24 weeks old (bodyweight: about 2.5 kg), talaporfin sodium solution (concentration: 5 mg/ml) was intravenously injected from the brachial vein in an amount of 2.5 mg/kg bodyweight of the cock, in terms of the amount of talaporfin sodium. Immediately after the intravenous injection, the cock's comb was irradiated with a diode laser (wavelength: 664.5nm ± 2nm, irradiation power density: 127mW/cm², energy density: 25J/cm² or 50J/cm²). As an experimental group, (i) PDT-performed group was made, and as control groups, (ii) a group to which only the intravenous injection of talaporfin sodium was performed, and (iii) a group to which only laser irradiation was performed, were made. The mean plasma talaporfin sodium level at the end of the treatment was also measured.

Morphological changes during 7 days after the treatment were visually observed, and the cocks were subjected to autopsy 7 days after the treatment. Tissue preparations were stained by hematoxylin eosin staining by a conventional method, and as required, PTAH staining (fibrin staining) and AZAN staining (collagen fiber staining) were added.

### Results

### Plasma Talaporfin Sodium Level

The mean plasma talaporfin sodium level at the end of treatment was 2.3 µg/mL.

### Visual Findings

In the PDT-performed group, decoloration reaction was observed at the site coincident with the irradiated site. Decoloration reaction was not observed in the group to which only the laser irradiation or only the drug administration was performed.

### Histological Findings

In the PDT-performed group, in the hematoxylin eosin staining, disappearance of vasodilated blood vessels in the dermis was observed in the site coincident with the irradiated site. In the irradiated site, the continuity of the stratum corneum epidermidis was maintained, and the stratum basale epidermidis was also conserved. In the highly magnified image in the dermis, disappearance of vascular endothelial cells was observed. Hyperplasia of collagen fibers in the stroma or the like was not observed. No changes were observed in the group to which only the laser irradiation or only the drug administration was performed.

### Discussion

From the experimental results, it was proved that vascular endothelial cells were selectively destructed without destructing the epidermis components by the PDT using talaporfin sodium. From the actually measured value (Example 2 below) of the plasma talaporfin sodium level in human, the amount of talaporfin sodium needed for attaining the above-described plasma talaporfin sodium level is 0.2 mg/kg. Thus, the amount of talaporfin sodium intravenously administered this time was 1/5 of the dose of 1.0 mg/kg generally administered in the therapies of malignant tumors, so that it was suggested that the therapy of cutaneous vascular lesions can be attained with a very small dose. Therefore, the longer shading time which is a side effect observed in the conventional PDT is thought to be able to be shortened. The wavelength of the laser used was 664.5 nm which was longer than 585 nm and 595 nm that are the wavelengths of the conventionally used dye lasers, so that the laser can reach a deeper portion. By using PDT, it was suggested that lesions at a deeper portion which could not be cured by the conventional therapies may possibly be treated. Further, the PDT is applicable even when the diameter of the blood vessel is large, so that varices may also be treated.

The principle of the conventional therapies to cutaneous vascular lesions is to selectively destruct the target cells by utilizing the conductive heat generated by irradiating the target cells in a living body with a laser having a high energy and having a wavelength coincident with the absorption wavelength of the target cells. In the PDT, a photosensitizer is applied to the target cells, and the target cells are irradiated with a laser having a low energy and having the wavelength coincident with that of the absorption wavelength of the drug, thereby to selectively destruct the target cells. Thus, PDT is very excellent in the respect that the maximum effect can be obtained in a living body with a low light energy. Therefore, to perform the PDT using talaporfin sodium is to change the concept of the conventional therapies of cutaneous vascular lesions.

### Example 2

### Safety Tests to Human

To 2 normal individuals (Subject A: normal male of 33 years old, Subject B: normal male of 42 years old), talaporfin sodium was intravenously administered at a dose of 0.2 mg/kg. After the administration, the skin of the upper arm was irradiated with a diode laser (irradiation conditions: irradiation energy of 25 J/cm² and 50 J/cm² at an energy density of 127 mW/cm², diameter of irradiated spots: 1 cm in both spots). Subject A was followed up for 2 weeks and Subject B was followed up for 2 months.

From immediately after the PDT, slight edema was observed in the irradiated sites and vicinities thereof. Up to 2 days after the treatment, slight erythema was observed in the sites coincident with the irradiated sites. At one week after the treatment, the erythema had been disappeared. In both subjects, both of the sites irradiated with the lasers having the energies of 25 J/cm² and 50 J/cm², respectively, were cured to such a degree that the irradiated sites could not be distinguished, and side effects such as pigmentation and depigmentation were not observed. In Subject B, at two months from the treatment, the irradiated sites could not be distinguished from the normal areas, and no side effects were observed. From these results, it was proved that dermatopathy does not occur even if the PDT using the drug for treating a vascular lesion according to the present invention is performed.

Further, it was confirmed from the change with time of the plasma drug level measured after the PDT that the plasma level at which the photosensitive reaction no longer occurs is attained at 24 to 48 hours from the intravenous injection. This period of time is almost coincident with the period of time deduced from the model of pharmacokinetics, so that the correctness of the above-described estimated required shading time was confirmed. Industrial Availability

By using the drug for treating a vascular lesion in the skin or subcutaneous soft tissue by light irradiation according to the present invention, the decoloration of the cutaneous vascular lesion irradiated with light is prominent, disappearance of vascular endothelial cells is observed, and yet the continuity of stratum corneum epidermidis is maintained and the stratum basale epidermidis is also conserved. Further, the shading time of this drug may be about 2 to 3 days. Therefore, the drug for treating a vascular lesion in the skin or subcutaneous soft tissue is very effective for the therapies of vascular lesions in the skin or subcutaneous soft tissue, represented by, capillary hemangioma, strawberry hemangioma, cavernous hemangioma, teleangiectasia and arteriovenous malformation. Further, by the diagnostic agent according to the present invention, the two-dimensional and further the three-dimensional extent of the vascular lesion in the skin or subcutaneous soft tissue can be diagnosed, which lesion cannot be observed from the skin surface.

## Claims

1. A drug for treating a vascular lesion in skin or subcutaneous soft tissue by light irradiation, said drug comprising as an effective ingredient mono-L-aspartyl chlorin e6 or mono-L-glutamyl chlorin e6, or a pharmaceutically acceptable salt thereof.

2. The drug according to claim 1, wherein said effective ingredient is mono-L-aspartyl chlorin e6 or an alkaline metal salt thereof.

3. The drug according to claim 2, wherein said effective ingredient is tetrasodium salt of mono-L-aspartyl chlorin e6.

4. The drug according to any one of claims 1 to 3, wherein said vascular lesion is capillary hemangioma, strawberry hemangioma, cavernous hemangioma, teleangiectasia or arteriovenous malformation.

5. The drug according to any one of claims 1 to 3, which is a drug for treating vascular lesion in skin.

6. The drug according to claim 5, wherein said vascular lesion in skin is capillary hemangioma, strawberry hemangioma or cavernous hemangioma.

7. The drug according to any one of claims 1 to 4, wherein said light irradiation is laser irradiation.

8. The drug according to claim 5 or 6, wherein said light irradiation is laser irradiation.

9. Use of mono-L-aspartyl chlorin e6 or mono-L-glutamyl chlorin e6, or a pharmaceutically acceptable salt thereof for the production of a drug for treating vascular lesion in skin or subcutaneous soft tissue by light irradiation.

10. A method for treating a vascular lesion in skin or subcutaneous soft tissue by light irradiation, said method comprising the steps of administering to a patient suffering from said vascular lesion in skin or subcutaneous soft tissue mono-L-aspartyl chlorin e6 or mono-L-glutamyl chlorin e6, or a pharmaceutically acceptable salt thereof; and then irradiating said vascular lesion with light.

11. A diagnostic agent for a vascular lesion in skin or subcutaneous soft tissue, comprising as an effective ingredient mono-L-aspartyl chlorin e6 or mono-L-glutamyl chlorin e6, or a pharmaceutically acceptable salt thereof.

12. Use of mono-L-aspartyl chlorin e6 or mono-L-glutamyl chlorin e6, or a pharmaceutically acceptable salt thereof for the production of a diagnostic agent for a vascular lesion in skin or subcutaneous soft tissue.

13. A method for diagnosing a vascular lesion, said method comprising the steps of administering to a patient suffering from said vascular lesion in skin or subcutaneous soft tissue mono-L-aspartyl chlorin e6 or mono-L-glutamyl chlorin e6, or a pharmaceutically acceptable salt thereof; then irradiating said vascular lesion with light; and measuring fluorescence from said vascular lesion.
